## Europäisches Patentamt

## European Patent Office

## Office européen des brevets

(19)

(11) Veröffentlichungsnummer: **0 033 763**
**B1**

(12) **EUROPÄISCHE PATENTSCHRIFT**

(45) Veröffentlichungstag der Patentschrift:
31.08.83

(21) Anmeldenummer: **80106975.8**

(22) Anmeldetag: **12.11.80**

(51) Int. Cl.³: **C 07 D 303/04,** C 07 D 301/16

(54) Verfahren zur Herstellung von 1,2-Epoxy-5,9-cyclododecadien.

(30) Priorität: **26.01.80 DE 3002838**

(43) Veröffentlichungstag der Anmeldung:
**19.08.81 Patentblatt 81/33**

(45) Bekanntmachung des Hinweises auf die Patenterteilung:
**31.08.83 Patentblatt 83/35**

(84) Benannte Vertragsstaaten:
**AT BE CH FR GB IT LI NL SE**

(56) Entgegenhaltungen:
**DE-A-962 073**
**DE-A-1 568 016**
**DE-A-1 768 530**
**DE-C-1 058 987**
**SU-A-149 420**

**Houben-Weyl 4. Auflage, Band VI/3, Seite 391 (1965)**

(73) Patentinhaber: **Degussa Aktiengesellschaft,**
**Weissfrauenstrasse 9, D-6000 Frankfurt am Main 1 (DE)**

(72) Erfinder: **Käbisch, Gerhard, Dr., Palmstrasse 1,**
**D-7888 Rheinfelden 5 (DE)**
Erfinder: **Trübe, Rudolf, Ernst-Reuter-Strasse 22,**
**D-7888 Rheinfelden (DE)**
Erfinder: **Wittmann, Hans, Liebenbergstrasse 12,**
**D-7888 Rheinfelden 8 (DE)**
Erfinder: **Raupach, Siegfried, Langentalstrasse 24,**
**D-7888 Rheinfelden (DE)**
Erfinder: **Malitius, Horst, Blümleacker 5,**
**D-7888 Rheinfelden (DE)**

## Verfahren zur Herstellung von 1,2-Epoxy-5,9-cyclododecadien

Die Erfindung betrifft ein Verfahren zur Herstellung von 1,2-Epoxy-5,9-cyclododecadien durch Umsetzung von 1,5,9-Cyclododecatrien mit in situ aus Ameisensäure und Wasserstoffperoxid gebildeter Perameisensäure.

Es ist bereits bekannt, aus 1,5,9-Cyclododecatrien mittels in situ gebildeter Perameisensäure das Monoepoxid herzustellen (DE-PS 1 058 987, Beispiel 9). Bei dem bekannten Verfahren wird das 1,5,9-Cyclododecatrien zusammen mit etwa 1,25 Mol pro Mol eines Wasserstoffperoxids mit einer Konzentration von 25 Gewichtsprozent vorgelegt. Zu der gerührten Mischung werden bei einer Temperatur unterhalb von 35 °C etwa 0,6 Mol Ameisensäure pro Mol 1,5,9-Cyclododecatrien zulaufen lassen. Nach 48 Stunden Rühren beträgt der Umsatz jedoch nur 60%.

Das erfindungsgemässe Verfahren ist dadurch gekennzeichnet, dass man das 1,5,9-Cyclododecatrien zusammen mit Ameisensäure einer Konzentration von mindestens 40 Gewichtsprozent in einer Menge zwischen 0,15 und 0,5 Mol pro Mol eingesetztem 1,5,9-Cyclododecatrien vorlegt und bei einer Temperatur zwischen 40 °C und 90 °C Wasserstoffperoxid einer Konzentration von mindestens 30 Gewichtsprozent in einer Menge von mindestens 1,05 Mol pro Mol eingesetztem 1,5,9-Cyclododecatrien während eines Zeitraumes von mindestens 3 Stunden und weniger als 8 Stunden zudosiert.

Das 1,5,9-Cyclododecatrien kann nach dem erfindungsgemässen Verfahren innerhalb relativ kurzer Reaktionszeiten mit hohem Umsatz zu dem entsprechenden Monoepoxid, dem 1,2-Epoxy-5,9-cyclododecadien, umgesetzt werden.

Zur Durchführung des erfindungsgemässen Verfahrens wird das 1,5,9-Cyclododecatrien zusammen mit Ameisensäure einer Konzentration von mindestens 40 Gewichtsprozent, vorzugsweise von 70 bis 100 Gewichtsprozent, vorgelegt. Die Ameisensäure wird in einer Menge zwischen 0,15 und 0,5 Mol pro Mol eingesetztem 1,5,9-Cyclododecatrien angewandt. Bevorzugt werden 0,25 bis 0,4 Mol verwendet.

Die Epoxydierung wird bei Temperaturen zwischen 40 °C und 90 °C, vorzugsweise zwischen 50 °C und 70 °C, vorgenommen. Dabei muss, insbesondere bei grösseren Ansätzen, für Möglichkeiten zu einer ausreichenden Abführung der freiwerdenden Reaktionswärme gesorgt werden. Das erfindungsgemässe Verfahren wird in Abwesenheit von Lösungsmitteln und von Katalysatoren für die Bildung der Perameisensäure aus der Ameisensäure und dem Wasserstoffperoxid durchgeführt.

Zu dem vorgelegten Gemisch aus 1,5,9-Cyclododecatrien und Ameisensäure wird unter Rühren Wasserstoffperoxid einer Konzentration von mindestens 30 Gewichtsprozent, vorzugsweise zwischen 35 und 70 Gewichtsprozent, in einer Menge von mindestens 1,05 Mol pro Mol eingesetztem 1,5,9-Cyclododecatrien während eines Zeitraumes von mindestens 3 Stunden und weniger als 8 Stunden zudosiert. Vorzugsweise wird das Wasserstoffperoxid in einer Menge zwischen 1,2 und 1,55 Mol pro Mol eingesetztem 1,5,9-Cyclododecatrien angewandt. Die Dosierung des Wasserstoffperoxids wird vorteilhafterweise so bemessen, dass in der wässrigen Phase mindestens 5 Stunden lang, vorzugsweise aber während der gesamten Dauer der Umsetzung, eine zwischen 30 und 70 Gewichtsprozent liegende Konzentration an Wasserstoffperoxid aufrecht erhalten wird.

Das erfindungsgemässe Verfahren ist auch in grosstechnischen Produktionsanlagen gefahrlos durchführbar, wenn einige sicherheitstechnische Massnahmen ergriffen werden. So können z.B. automatische Sicherheitsventile vorgesehen werden, die sich öffnen und das Reaktionsgemisch mit Wasser verdünnen, wenn die Mischorgane ausfallen, die Menge des durch Zersetzung von Wasserstoffperoxid oder Perameisensäure gebildeten Abgases einen vorgegebenen Grenzwert überschreitet oder die Temperatur merklich über 90 °C anzusteigen droht.

Die Umsetzung zwischen dem 1,5,9-Cyclododecatrien und der in situ gebildeten Perameisensäure wird vorteilhafterweise drucklos durchgeführt. Sie erfordert im allgemeinen eine Reaktionszeit zwischen 5 und 10 Stunden. Nach beendeter Epoxydierungsreaktion wird im Normalfall die wässrige Phase von der organischen Epoxidphase getrennt. Zur weiteren Reinigung empfiehlt es sich, die organische Phase von darin noch gelösten Wasserstoffperoxid- und Ameisensäure-Anteilen zu befreien. Bevorzugt wird hierzu ein zweimal hintereinander vorgenommenes Auswaschen mit Wasser, wobei das Volumenverhältnis zwischen der organischen Phase und dem Waschwasser jeweils etwa 10 : 1 bis 2 : 1 beträgt. Wegen der geringen Wasserlöslichkeit des gebildeten Monoepoxids kann das Auswaschen auch bei erhöhten Temperaturen vorgenommen werden. Die organische Phase muss daher nach beendeter Epoxydierungsreaktion nicht erst abgekühlt werden.

Das 1,2-Epoxy-5,9-cyclododecadien ist ein wertvolles Zwischenprodukt für weitere Synthesen. Für die Weiterverarbeitung soll es im allgemeinen in möglichst reiner Form eingesetzt werden. Das beim erfindungsgemässen Verfahren anfallende rohe Reaktionsprodukt kann sehr leicht von nicht umgesetztem 1,5,9-Cyclododecatrien befreit werden, indem man das letztere bei vermindertem Druck abtreibt. Das zurückbleibende Sumpfprodukt ist dann im allgemeinen für die Weiterverarbeitung ausreichend rein. Erforderlichenfalls kann es auch durch fraktionierte Destillation bei vermindertem Druck weiter gereinigt werden. Die nach der Abtrennung der organischen Phase hinterbleibende abgereicherte wässrige Phase kann in an sich bekannter Weise zur Rückgewinnung des enthaltenen Wasserstoffperoxids aufgearbeitet werden.

Beispiel 1

In einem 1 1-Dreihalskolben mit Rührer, Rückflusskühler, Tropftrichter und Thermometer werden
405,5 g (2,5 Mol) 1,5,9-Cyclododecatrien,
28,8 g (0,625 Mol) Ameisensäure (98 Gewichtsprozent) und
0,3 g des Natriumsalzes der Hydroxyethandiphosphonsäure (als Stabilisator für das Wasserstoffperoxid) vorgelegt.

Unter intensiver Rührung und Kühlung zur Abführung der freiwerdenden Reaktionswärme werden im Verlaufe von 5 Stunden
145,5 g (3,0 Mol) Wasserstoffperoxid (70 Gewichtsprozent)
zudosiert.

Während der Zudosierung des Wasserstoffperoxids und einer anschliessenden Nachreaktionszeit von weiteren 2 Stunden wird die Temperatur im Inneren des Rührkolbens zwischen 46 und 54 °C gehalten.

Nach beendeter Umsetzung wird die wässrige Phase in einem Scheidetrichter abgetrennt und die organische Phase zweimal hintereinander mit je 100 ml Wasser nachgewaschen. Aus dem angefallenen Rohprodukt werden bei vermindertem Druck (12 Torr) bis zu einer Maximaltemperatur von 125 °C 126,5 g nicht umgesetztes 1,5,9-Cyclododecatrien abgetrieben. Der Umsatz beträgt demnach 68,8% der Theorie. Das zurückbleibende Sumpfprodukt wiegt 306,5 g und besteht zu ca. 99% aus dem Monoepoxid 1,2-Epoxy-5,9-cyclododecadien, das die folgenden physikalischen Eigenschaften aufweist:

$$n\,_D^{20} : 1,5060$$

$$Kp_{12} : 130 \text{ bis } 132\,°C$$
$$Kp_{0,1} : 74 \text{ bis } 76\,°C.$$

Beispiel 2

In einer Apparatur gemäss Beispiel 1 werden vorgelegt
162,3 g (1,0 Mol) 1,5,9-Cyclododecatrien
18,4 g (0,4 Mol) Ameisensäure (98 Gewichtsprozent) und
0,1 g Stabilisator (wie in Beispiel 1).

Unter kräftigem Rühren werden bei 65 °C im Verlaufe von 4 Stunden 96,0 g (1,2 Mol) Wasserstoffperoxid (42,5 Gewichtsprozent) gleichmässig zudosiert. Der Ansatz wird noch weitere 1,5 Stunden bei 65 °C gerührt und danach im Scheidetrichter die wässrige Phase abgetrennt. Nach dem Auswaschen der organischen Phase mit Wasser (zweimal mit je 50 ml) zeigt diese nachstehende Zusammensetzung:

65 Gewichtsprozent 1,2-Epoxy-5,9-cyclododecadien
34 Gewichtsprozent nicht umgesetztes 1,5,9-Cyclododecatrien
1 Gewichtsprozent hochsiedende Nebenprodukte.

Beispiel 3

In einer Apparatur gemäss Beispiel 1 werden vorgelegt

162,3 g (1,0 Mol) 1,5,9-Cyclododecatrien
19,5 g (0,2 Mol) Ameisensäure (47,2 Gewichtsprozent) und
0,1 g Stabilisator (wie in Beispiel 1).

Unter kräftigem Rühren werden bei 50 °C im Verlaufe von 4 Stunden 48,0 g (1,2 Mol) Wasserstoffperoxid (85 Gewichtsprozent) gleichmässig zudosiert. Der Ansatz wird noch weitere 1,5 Stunden bei 50 °C gerührt und danach die organische Phase wie in Beispiel 2 gewaschen. Die gewaschene organische Phase zeigt nachstehende Zusammensetzung:

68 Gewichtsprozent 1,2-Epoxy-5,9-cyclododecadien
31 Gewichtsprozent nicht umgesetztes 1,5,9-Cyclododecatrien
1 Gewichtsprozent hochsiedende Nebenprodukte.

### Patentansprüche

1. Verfahren zur Herstellung von 1,2-Epoxy-5,9-cyclododecadien durch Umsetzung von 1,5,9-Cyclododecatrien mit in situ aus Ameisensäure und Wasserstoffperoxid gebildeter Perameisensäure, dadurch gekennzeichnet, dass man das 1,59-Cyclododecatrien zusammen mit Ameisensäure einer Konzentration von mindestens 40 Gewichtsprozent in einer Menge zwischen 0,15 und 0,5 Mol pro Mol eingesetztem 1,5,9-Cyclododecatrien vorlegt und bei einer Temperatur zwischen 40 °C und 90 °C Wasserstoffperoxid einer Konzentration von mindestens 30 Gewichtsprozent in einer Menge von mindestens 1,05 Mol pro Mol eingesetztem 1,5,9-Cyclododecatrien während eines Zeitraumes von mindestens 3 Stunden und weniger als 8 Stunden zudosiert.

2. Verfahren nach Anspruch 1, dadurch gekennzeichnet, dass man eine Ameisensäure mit einer Konzentration von 70 bis 100 Gewichtsprozent einsetzt.

3. Verfahren nach Anspruch 1 oder 2, dadurch gekennzeichnet, dass man die Ameisensäure in einer Menge zwischen 0,25 und 0,40 Mol pro Mol eingesetztem 1,5,9-Cyclododecatrien einsetzt.

4. Verfahren nach einem der Ansprüche 1 bis 3, dadurch gekennzeichnet, dass man Wasserstoffperoxid mit einer Konzentration von 35 bis 70 Gewichtsprozent einsetzt.

5. Verfahren nach einem der Ansprüche 1 bis 4, dadurch gekennzeichnet, dass man das Wasserstoffperoxid in einer Menge zwischen 1,2 und 1,55 Mol pro Mol eingesetztem 1,5,9-Cyclododecatrien einsetzt.

### Revendications

1. Procédé pour la fabrication de 1,2-époxy-5,9-cyclododécadiène, par réaction de 1,5,9-cyclododécatriène avec de l'acide performique, formé insitu à partir d'acide formique et de peroxyde d'hydrogène, procédé caractérisé en ce qu'on met en présence tout d'abord le 1,5,9-cyclododécatriène avec l'acide formique d'une concentration égale à au moins 40% en poids, en une quantité comprise entre 0,15 et 0,5 mole par mole de 1,5,9-cyclodo-décatriène mis en œuvre, et on ajoute, à une température comprise entre 40 °C et 90 °C, progressivement pendant une durée d'au moins 3 heures et inférieure à 8 heures, du peroxyde d'hydrogène, de concentration d'au moins 30% en poids, en une quantité égale à au moins 1,05 mole par mole de 1,5,9-cyclododécatriène mis en œuvre.

2. Procédé suivant la revendication 1, caractérisé en ce qu'on met en réaction un acide formique ayant une concentration comprise entre 70 et 100%.

3. Procédé suivant l'une des revendications 1 et 2, caractérisé en ce que l'acide formique est introduit en une quantité comprise entre 0,25 et 0,40 mole par mole de 1,5,9-cyclododécatriène mis en œuvre.

4. Procédé selon l'une quelconque des revendications 1 à 3, caractérisé en ce qu'on introduit du peroxyde d'hydrogène ayant une concentration de 35 à 70% en poids.

5. Procédé suivant l'une quelconque des revendications 1 à 4, caractérisé en ce que le peroxyde d'hydrogène est introduit en une quantité comprise entre 1,2 et 1,55 mole par mole de 1,5,9-cyclododécatriène mis en œuvre.

**Claims**

1. A process for the production of 1,2-epoxy-5,9-cyclododecadiene by the reaction of 1,5,9-cyclododecatriene with performic acid formed in situ from formic acid and hydrogen peroxide, characterised in that the 1,5,9-cyclododecatriene is introduced together with formic acid of a concentration of at least 40% by weight in a quantity of from 0.15 to 0.5 mols per mol of 1,5,9-cyclododecatriene which is used, and hydrogen peroxide of a concentration of at least 30% by weight is metered in at a temperature of from 40 to 90 °C in a quantity of at least 1.05 mols per mol of 1,5,9-cyclododecatriene which is used, over a period of at least 3 hours and less than 8 hours.

2. A process according to claim 1, characterised in that a formic acid of a concentration of from 70 to 100% by weight is used.

3. A process according to claim 1 or 2, characterised in that the formic acid is used in a quantity of from 0.25 to 0.40 mols per mol of 1,5,9-cyclododecatriene which is used.

4. A process according to one of claims 1 to 3, characterised in that hydrogen peroxide of a concentration of from 35 to 70% by weight is used.

5. A process according to one of claims 1 to 4, characterised in that the hydrogen peroxide is used in a quantity of from 1.2 to 1.55 mols per mol of 1,5,9-cyclododecatriene which is used.